(19) Europäisches Patentamt
European Patent Office
Office européen des brevets

(11) Veröffentlichungsnummer: **0 021 160 A1**

# (12) EUROPÄISCHE PATENTANMELDUNG

(21) Anmeldenummer: **80103110.5**

(22) Anmeldetag: **04.06.80**

(51) Int. Cl.³: **C 07 C 61/04**, C 07 C 51/29, C 07 C 62/18, C 07 C 51/09 // C07C148/00, C07C149/46, C07C69/757, C07C67/343

(30) Priorität: **12.06.79 DE 2923773**

(43) Veröffentlichungstag der Anmeldung: **07.01.81 Patentblatt 81/1**

(84) Benannte Vertragsstaaten: **BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Maurer, Fritz, Dr., Roeberstrasse 8, D-5600 Wuppertal 1 (DE)**

## (54) Verfahren zur Herstellung von trans-3,3-Dimethyl-cyclopropan-1,2-dicarbonsäure.

(57) Trans-3,3-Dimethylcyclopropan-1,2-dicarbonsäure, werden hergestellt aus 2-Acetyl-3,3-dimethylcyclopropan-1-carbonsäureester, in einer ersten Stufe durch Verseifung mit wässrig-alkoholischen Alkalilaugen und, nach Abdestillieren des Alkohols, in einer zweiten Stufe durch Umsetzung der wäßrigen Alkalisalzlösung mit wäßrigen Lösungen von Alkalihypochloriten.

EP 0 021 160 A1

0021160

BAYER AKTIENGESELLSCHAFT
Zentralbereich
Patente, Marken und Lizenzen

5090 Leverkusen, Bayerwerk
Rt-by
Typ IVa/ZP

# Verfahren zur Herstellung von trans-3,3-Dimethyl-cyclopropan-1,2-dicarbonsäure

Die Erfindung betrifft ein neues Verfahren zur Herstellung der bekannten trans-3,3-Dimethylcyclopropan-1,2-dicarbonsäure (trans-Caronsäure), welche als Zwischenprodukt zur Herstellung von pestiziden Wirkstoffen verwendet werden kann.

Es ist bereits bekannt geworden, daß man trans-3,3-Dimethyl-cyclopropan-1,2-dicarbonsäure (trans-Caronsäure) erhält, wenn man Fumarsäure-dimethylester mit Dimethyldiazomethan bei Temperaturen zwischen 0 und 5°C umsetzt, das dabei entstehende 1:1-Addukt aus diesen Verbindungen in Gegenwart von Kupferpulver als Katalysator auf 160-170°C erhitzt, bis die unter Stickstoffeliminierung ablaufende Bildung von trans-Caronsäure-dimethylester abgelaufen ist, und anschließend eine Esterverseifung durchführt (vergleiche J. Am. Chem. Soc. 82 (1960), 5255). Diese Synthesemethode ist mit den durch die Verwendung von Dimethyldiazomethan bedingten Sicherheitsrisiken behaftet; die Ausbeute an trans-Caronsäure ist dabei in der Regel unbefriedigend.

Weiter ist bekannt geworden, daß cis- bzw. trans-Caronsäure-dimethylester durch Umsetzung von Maleinsäure- bzw. Fumarsäure-dimethylester mit Diphenylsulfoniumisopropylid bei -70°C in guten Ausbeuten synthetisiert werden können; die freien Säuren erhält man daraus nach üblichen Verseifungsmethoden (vergleiche J. Am. Chem. Soc. 89 (1967), 3912-3914; ibid. 95 (1973), 3970-3976). Das bei dieser Synthesemethode als Reaktionskomponente einzusetzende Diphenylsulfonium-isopropylid wird durch Umsetzung von Diphenyl-äthyl-sulfonium-tetrafluoroborat mit Lithium-di-isopropylamid, anschließende Alkylierung mit Methyliodid und erneute Umsetzung mit Lithium-di-isopropylamid bei -70°C hergestellt.

An Stelle von Diphenylsulfonium-isopropylid kann auch Triphenylphosphonium-isopropylid zur Synthese von trans-3,3-Dimethyl-cyclopropan-1,2-dicarbonsäureestern, ausgehend von Maleinsäure- und/oder Fumarsäureestern verwendet werden (vergleiche Tetrahedron Lett. 1978, 1847-1850).

Die Umsetzungen mit den genannten Yliden werden wegen deren Empfindlichkeit gegen Luftsauerstoff und Feuchtigkeit unter einer Inertgasatomosphäre und unter Verwendung sorgfältig getrockneter Lösungsmittel durchgeführt. Das gleiche gilt auch für die Herstellung der Ylide; hierfür werden sehr starke Basen wie z.B. Butyllithium und/oder Lithium-di-isopropylamid benötigt.

Die oben erläuterten bekannten Synthesemethoden sind wegen der aufwendigen Reaktionsführung, insbesondere auch bei der Herstellung von Vorprodukten, für die Herstellung von 3,3-Dimethyl-cyclopropan-1,2-dicarbonsäure bzw. von deren Estern im industriellen Maßstab wenig geeignet.

Es wurde ein Verfahren zur Herstellung von trans-3,3-Dimethylcyclopropan-1,2-dicarbonsäure der Formel (I) gefunden,

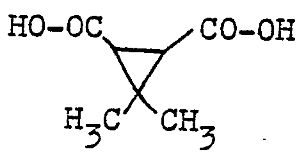

(I)

welches dadurch gekennzeichnet ist, daß man 2-Acetyl-3,3-Dimethyl-cyclopropan-1-carbonsäureester der Formel (II)

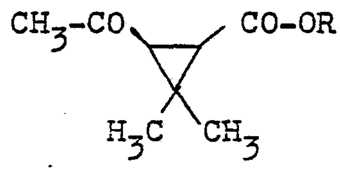

(II)

in welcher
R für Alkyl mit 1 bis 4 Kohlenstoffatomen steht,

in einer ersten Stufe durch Umsetzung mit wässrig-alkoholischen Alkalilaugen bei Temperaturen zwischen 2o und 15o°C zu einem Alkalimetallsalz von 2-Acetyl-3,3-dimethyl-cyclopropan-1-carbonsäure der Formel III

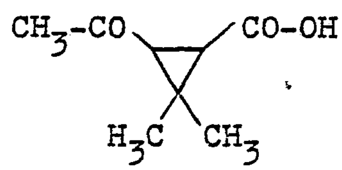

(III)

verseift und nach Abdestillieren des Alkohols in einer zweiten Stufe die wässrige Lösung der Alkalisalze von 2-Acetyl-3,3-dimethyl-cyclopropan-1-carbonsäure (III) mit wäßrigen Lösungen von Alkalihypochloriten, bei Temperaturen zwischen 0 und 100°C umsetzt, und daraus die freie Säure der Formel (I) in üblicher Weise isoliert.

Überraschenderweise kann nach dem neuen Verfahren, welches weit weniger technischen Aufwand als bekannte Synthesemethoden erfordert, trans-3,3-Dimethyl-cyclopropan-1,2-dicarbonsäure mit Hilfe einfacher und billiger Reagentien in hoher Reinheit und mit sehr guten Ausbeuten hergestellt werden.

Verwendet man als Ausgangsverbindung beispielsweise 2-Acetyl-3,3-dimethyl-cyclopropan-1-carbonsäure-äthylester, so kann die Reaktion mit wässrig-alkoholischer Natronlauge und die anschließende Umsetzung mit Natriumhypochlorit, wonach das Produkt beispielsweise durch Ansäuern mit Salzsäure ausgefällt wird, durch folgendes Formelschema skizziert werden:

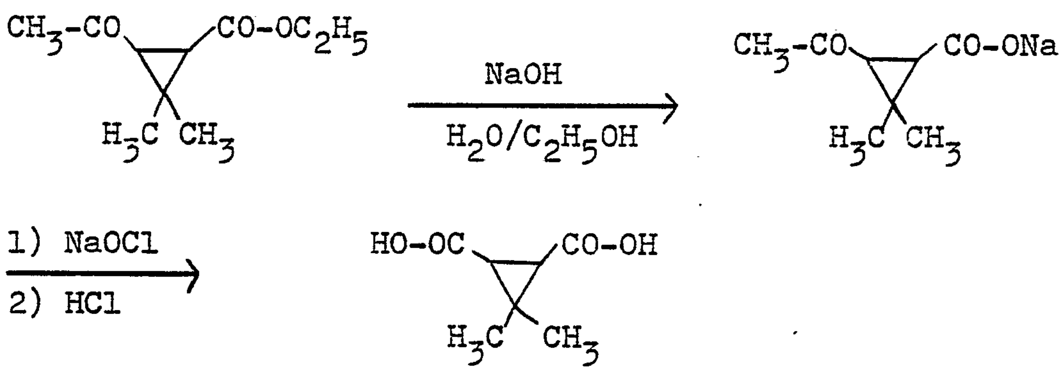

Als Beispiele für die Ausgangsverbindungen der Formel (II) seien genannt:
2-Acetyl-3,3-dimethyl-cyclopropan-1-carbonsäuremethylester, -äthylester, -n-propylester, -iso-propylester, -n-butylester, -iso-butylester, -sek.-butylester und -tert.-butylester.

2-Acetyl-3,3-dimethyl-cyclopropan-1-carbonsäureäthylester ist bereits in der Literatur beschrieben (vergleiche J.Org.Chem. 32 (1967), 3351-3355). Diese Verbindung und ihre Homologen der Formel (II) können nach einem im Prinzip bekannten Verfahren hergestellt werden. Man erhält sie, indem man 4-Methyl-pent-3-en-2-on ("Mesityloxid") der Formel IV

$$CH_3-CO-CH=\overset{\displaystyle CH_3}{\overset{|}{C}}-CH_3 \qquad (IV)$$

mit Dimethylsulfuranyliden-essigsäureestern der Formel V

$$\begin{matrix} H_3C \\ H_3C \end{matrix} \!\!> S=CH-CO-OR \qquad (V)$$

in welcher
R   die oben angegebene Bedeutung hat,

gegebenenfalls unter Verwendung eines Verdünnungsmittels wie z.B. Benzol, bei Temperaturen zwischen

2o und loo°C umsetzt und destillativ aufarbeitet.

Als Beispiele für die Vorprodukte der Formel (V) seien genannt:
Dimethylsulfuranyliden-essigsäure-methylester, -äthylester, -n-propylester, -iso-propylester, -n-butylester, -iso-butylester, -sek.-butylester und -tert.-butylester.

Dimethylsulfuranyliden-essigsäureäthylester ist bereits bekannt (vergleiche J. Org. Chem. 32 (1967), 3351-3355). Man erhält diese Verbindung und ihre Homologen der Formel (V), indem man Carbalkoxymethyl-dimethylsulfoniumhalogenide der Formel VI

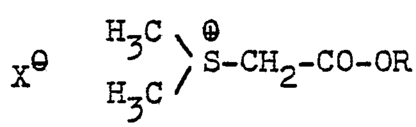 (VI)

in welcher
R die oben angegeben Bedeutung hat und
X für Chlor oder Brom steht,

in einem organischen, mit Wasser nicht mischbaren Lösungsmittel wie z.B. Chloroform löst und mit einer wässrigen gesättigten Kaliumcarbonatlösung, welche ca. ein Moläquivalent eines Alkalihydroxids wie z.B. Natriumhydroxid enthält, bei Temperaturen zwischen lo und 2o°C intensiv verrührt und nach Abtrennen der organischen Phase hiervon das Lösungsmittel im Vakuum abdestilliert.

Als Beispiele für die Verbindungen der Formel (VI) seien genannt:
Carbomethoxymethyl-, Carbäthoxymethyl-, Carbo-n-propoxymethyl-, Carbo-iso-propoxymethyl-, Carbo-n-butoxymethyl-, Carbo-iso-butoxy-methyl-, Carbo-sek.-butoxy-methyl- und Carbo-tert.-butoxymethyl-dimethylsulfonium-bromid.

Carbäthoxymethyl-dimethylsulfoniumbromid ist bereits bekannt (vergleiche J.Org.Chem. 32 (1967), 3351-3355). Man erhält diese Verbindung und ihre Homologen der Formel (VI), indem man Halogenessigsäureester der Formel VII

$$X\text{-}CH_2\text{-}CO\text{-}OR \qquad \text{(VII)}$$

in welcher
R und X die oben angegebene Bedeutung haben,

mit Dimethylsulfid in einem organischen Lösungsmittel wie z.B. Aceton bei Temperaturen zwischen 0 und 50°C umsetzt und von den kristallin anfallenden Produkten absaugt.

Als Beispiele für die Halogenessigsäureester der Formel (VII), welches bekannte Verbindungen sind, seien genannt:
Bromessigsäure-methylester, -äthylester, -n-propylester, -iso-propylester, -n-butylester, -isobutylester, -sek.-butylester und -tert.-butylester.

Die erste Stufe des erfindungsgemäßen Verfahrens wird im allgemeinen unter Verwendung von Gemischen aus Wasser und Alkoholen als Verdünnungsmitteln durchgeführt. Als Beispiele für die zu verwendenden Alkohole seien genannt:
Methanol, Äthanol, n-Propanol, iso-Propanol, n-Butanol, iso-Butanol, sek.-Butanol und tert.-Butanol.

Vor der Durchführung der zweiten Verfahrensstufe werden die Alkohole weitgehend abdestilliert und die Reaktionsgemische gegebenenfalls mit Wasser wieder verdünnt.

Zur Verseifung der 2-Acetyl-3,3-dimethyl-cyclopropan-1-carbonsäureester werden wässrig-alkoholische Lösungen von Alkalihydroxiden, vorzugsweise von Natriumhydroxid oder Kaliumhydroxid, verwendet.

Als Reagentien für die Umwandlung von 2-Acetyl-3,3-dimethyl-cyclopropan-1-carbonsäure (bzw. von deren Salzen) in trans-3,3-Dimethyl-cyclopropan-1,2-dicarbonsäure (bzw. deren Salze) werden Alkali- oder Erdalkalihypochlorite, vorzugsweise Natrium-, Kalium- oder Calcium-hypochlorit eingesetzt. Die Verwendung technischer Hypochloritlösungen (z.B. von "Chlorlauge") ist ebenso gut möglich wie die Verwendung wässriger Lösungen von reinen Hypochloriten.

Die Reaktionstemperaturen liegen in der ersten Verfahrensstufe im allgemeinen zwischen 2o und 15o°C, vorzugsweise zwischen 5o und 12o°C; in der zweiten Stufe zwischen 0 und 1oo°C, vorzugsweise bei 2o bis 8o°C. Beide Stufen des erfindungsgemäßen Verfahrens werden im allgemeinen bei Normaldruck durchgeführt.

Zur Durchführung des erfindungsgemäßen Verfahrens werden in der ersten Stufe 1 Mol 2-Acetyl-3,3-dimethyl-cyclopropan-1-carbonsäureester der Formel (II) zu 1 bis 3 Mol, vorzugsweise 1,5 bis 2,5 Mol Alkalihydroxid in wässrig-alkoholischer Lösung gegeben und im oben angegebenen Temperaturbereich gehalten, bis die Verseifungsreaktion abgelaufen ist. Anschließend wird der Alkohol und teilweise das Wasser im Vakuum abdestilliert, das Reaktionsgemisch mit Wasser wieder verdünnt und mit Hypochloritlösung, welche wenigstens 3 Mol aktives Chlor je Mol eingesetztem 2-Acetyl-3,3-dimethyl-cyclopropan-1-carbonsäureester enthält, versetzt. Man rührt die Reaktionsmischung einige Stunden bei der oben angegebenen Temperatur, kühlt dann mit Eis ab, versetzt mit Hydrogensulfitlösung, bis das überschüssige Hypochlorit abreagiert hat und säuert mit einer Mineralsäure wie z.B. Salzsäure an.

Die auf diese Weise in kristalliner Form erhaltene trans-3,3-Dimethyl-cyclopropan-1,2-dicarbonsäure kann als Zwischenprodukt zur Herstellung von Insektiziden verwendet werden (vergleiche Pestic. Sci. 7 (1976), 492-498; Tetrahedron Lett. 1978, 1847-185o).

Beispiel

Darstellung von trans-3,3-Dimethyl-cyclopropan-1,2-dicarbonsäure:

Zu einer Lösung von 2oo g (3,6 Mol) Kaliumhydroxid in einem Gemisch aus 8oo ml Wasser und 8oo ml Äthanol gibt man 46o g 7o%igen 2-Acetyl-3,3-dimethyl-cyclopropancarbonsäureäthylester (dargestellt nach G.B.Payne, J.Org.Chem. 32 (1967), 3351-3355) und kocht die Mischung 17 Stunden unter Rückfluß. Dann destilliert man das Äthanol und einen Teil des Wassers im Vakuum ab. Die zurückbleibende Lösung wird zunächst mit 5oo ml Wasser verdünnt und dann ohne Kühlung mit 4,5 l Chlorlauge (ca. 2 Mol aktives Chlor pro Liter) versetzt. Die Temperatur steigt dabei auf ca. 5o°C an. Man rührt das Gemisch noch 2 Stunden bei 5o-6o°C, kühlt auf 1o°C ab und gibt Natriumhydrogensulfitlösung zu bis alle Chlorlauge zerstört ist. Dann stellt man durch Zugabe von konzentrierter Salzsäure auf pH 2 ein, rührt eine halbe Stunde bei 1o°C und saugt das ausgefallene Produkt ab. Man wäscht mit ca. 1,5 l Eiswasser nach und trocknet die Verbindung bei 6o°C im Vakuum. Man erhält auf diese Weise 26o g (93 % der Theorie) trans-3,3-Dimethyl-cyclopropan-1,2-dicarbonsäure mit dem Schmelzpunkt 214°C.

Patentansprüche

1. Verfahren zur Herstellung von trans-3,3-Dimethyl-cyclopropan-1,2-dicarbonsäure der Formel (I)

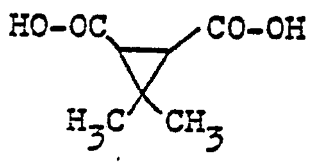

(I)

dadurch gekennzeichnet, daß man 2-Acetyl-3,3-dimethyl-cyclopropan-1-carbonsäureester der Formel (II)

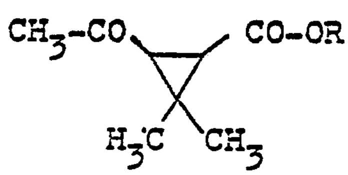

(II).

in welcher

R für Alkyl mit 1 bis 4 Kohlenstoffatomen steht,

in einer ersten Stufe durch Umsetzung mit wäßrigalkoholischen Alkalilaugen bei Temperaturen zwischen 20 und 150°C zu einem Alkalimetallsalz von 2-Acetyl-3,3-dimethyl-cyclopropan-1-carbonsäure der Formel (III)

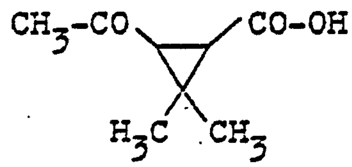

(III)

verseift und nach Abdestillieren des Alkohols in einer zweiten Stufe die wäßrige Lösung der Alkalisalze von 2-Acetyl-3,3-dimethyl-cyclopropan-1-carbonsäure (III) mit wäßrigen Lösungen von Alkalihypochloriten, bei Temperaturen zwischen 0 und 100°C umsetzt, und daraus die freie Säure der Formel (I) in üblicher Weise isoliert.

Europäisches Patentamt

# EUROPÄISCHER RECHERCHENBERICHT

0021160

Nummer der Anmeldung

EP 80 10 3110

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 605 398 (SUMITOMO)<br>* Seite 12, Zeile 1 - Seite 14, Zeile 9; Seite 22, Beispiel 2 *<br>-- | 1 |
| | DE - A - 2 621 833 (I.C.I.)<br>* Ansprüche 1-5 *<br>-- | 1 |
| | DE - A - 2 606 635 (BAYER)<br>* Seite 18 *<br>-- | 1 |
| | DE - A - 2 639 777 (THE WELLCOME FOUNDATION)<br>* Beispiele 8,14 *<br>---- | 1 |

KLASSIFIKATION DER ANMELDUNG (Int. Cl.³)

C 07 C 61/04
C 07 C 51/29
62/18
51/09//
C 07 C 148/00
149/46
69/757
67/343

RECHERCHIERTE SACHGEBIETE (Int. Cl.³)

C 07 C 61/04
51/29

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 18-09-1980 | LI VOTI |

EPA form 1503.1 06.78